(19) Europäisches Patentamt
European Patent Office
Office européen des brevets

(11) Veröffentlichungsnummer: **0 287 894 B1**

(12) **EUROPÄISCHE PATENTSCHRIFT**

(45) Veröffentlichungstag der Patentschrift: **09.12.92**

(51) Int. Cl.⁵: **C07D 305/12**

(21) Anmeldenummer: **88105486.0**

(22) Anmeldetag: **06.04.88**

# (54) Verfahren zur Verminderung des Ketentrimergehaltes in Diketen.

(30) Priorität: **16.04.87 CH 1489/87**

(43) Veröffentlichungstag der Anmeldung:
**26.10.88 Patentblatt 88/43**

(45) Bekanntmachung des Hinweises auf die Patenterteilung:
**09.12.92 Patentblatt 92/50**

(84) Benannte Vertragsstaaten:
**CH DE FR LI**

(56) Entgegenhaltungen:

**HELVETICA CHIMICA ACTA, vol 29, 1977, Basel.; L. TENUD et al "1,3-Cyclobutandionderivate aus Keten", Seiten 975-977**

**ORGANIC SYNTHESES, vol 21, 1941, New York; J.W. WILLIAMS et al "Ketene Dimer", Seiten 64-66**

**JOURNAL OF ORGANIC CHEMISTRY, vol 38, 1973, Washington, DC, US; H.H. WASSERMAN et al "Cyclobutenone Derivatives from Ethoxyacetylene "Seiten 1451-1455**

(73) Patentinhaber: **LONZA AG**
**Gampel/Wallis(CH)**

(72) Erfinder: **Bergamin, Renzo, Dr.-Ing.-Chem.**
**Haus Burg**
**Raron ( Wallis )(CH)**
Erfinder: **Ouittmann, Wilhelm, Dr.**
**Rottenstrasse 13**
**Visp ( Wallis )(CH)**

(74) Vertreter: **Weinhold, Peter, Dr. et al**
**Patentanwälte Dr. V. Schmied-Kowarzik**
**Dipl.-Ing. G. Dannenberg Dr. P. Weinhold Dr. D. Gudel Dipl.-Ing. S. Schubert Dr. P. Barz**
**Siegfriedstrasse 8**
**W-8000 München 40(DE)**

EP 0 287 894 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Verminderung des Ketentrimergehaltes in Roh-Diketen.

Es ist bekannt, dass sich bei der Dimerisierung von Keten neben Diketen als Hauptprodukt noch polymere Harze und ein Ketentrimer bilden. Dieses Ketentrimer wurde als 3-Acetoxycyclobut-2-en-1-on identifiziert und liegt in Anteilen bis ca. 5% im Diketen vor. Diese Produktenmischung wird erfindungsgemäß vorzugsweise eingesetzt.

Es ist weiter bekannt, dass dieses Ketentrimer beim Erhitzen über 50°C zu stark exothermen Reaktionen neigt [Helv.Chim. Acta 60 (1977) Nr.100, S.975 ff]. Diese Eigenschaft, beim Erhitzen unkontrollierte, exotherme Reaktionen einzugehen, macht das Ketentrimer zu einem Sicherheitsrisiko bei der Herstellung und Destillation des Diketens.

Aufgabe der vorliegenden Erfindung ist es, das Ketentrimer auf einfache Weise aus dem Roh-Diketen zu entfernen oder seinen Gehalt zu vermindern. Erreicht wird dies erfindungsgemäß dadurch, daß man Diketen mit einer dem Ketentrimer im Diketen entsprechenden Menge einer Verbindung der allgemeinen Formel

$R(XH)_n$

in welcher R = H, aliphatische Kohlenwasserstoffreste mit 1 bis 18 C-Atomen oder einen aromatischen Kohlenwasserstoffrest, X = Sauerstoff oder Schwefel, oder XH = eine COOH-Gruppe und n = 1 oder 2 bedeuten, versetzt, auf Temperaturen von 50 bis 130°C erwärmt und das Diketen abdestilliert.

Als Verbindung $R(XH)_n$ können Wasser, ein- oder mehrwertige Alkohole mit 1 bis 18 C-Atomen, ein- oder mehrwertige Phenole, Thiole, Polythiole oder Carbonsäuren verwendet werden. Solche sind beispielsweise:

| | |
|---|---|
| Alkohole: | Methanol, Ethanol, Propanol, Heptanol, Octadecanol, Diethylenglykol. |
| Phenole: | Phenol, Hydrochinon. |
| Thiole: | Butylthiol, Octadecanthiol. |
| Polythiole: | 1,3-Butyldithiol, 1,10-Decandithiol. |
| Carbonsäuren: | Ameisensäure, Essigsäure, Oxalsäure, Malonsäure, Bernsteinsäure. |

Vorzugsweise werden Alkohole mit 1 bis 6 C-Atomen im Molekül oder Phenol verwendet. Sofern R einen aromatischen Kohlenwasserstoffrest darstellt, kann er Phenyl bedeuten. Die Temperatur von 50 bis 130°C wird zweckmässig während 0,25 bis 5 Stunden gehalten. Danach kann das Diketen ohne Risiko destilliert werden.

Bei der Behandlung des Roh-Diketens mit einer Verbindung der Formel $R(XH)_n$ entstehen thermostabile Butencarbonsäurederivate der Formel

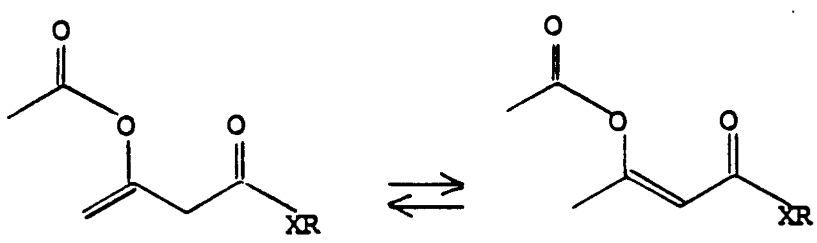

n = 1

oder

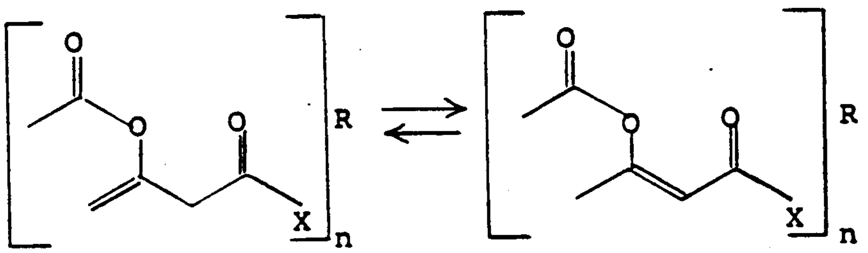

n = 2

Das Verfahren der Erfindung kann in einem Rührkessel im Batch-Verfahren oder kontinuierlich durchgeführt werden.

Beispiel 1

1 kg Diketen-Rohprodukt, das einen Gehalt von 4,2% Ketentrimer enthielt, wurde in einem thermostatisierbaren Rührkessel mit der äquivalenten Menge Methanol versetzt und während 65 Min. auf 60 bis 63 °C erwärmt. Nach dieser Behandlung war der Anteil an Ketentrimer unter die Nachweisgrenze gesunken. das praktisch ketentrimerfreie Roh-Diketen konnte nun mit stark erhöhter Sicherheit destilliert werden.

Beispiel 2

0,1 kg Diketen-Rohprodukt, das einen Gehalt von 4,3% Ketentrimer enthielt, wurde in einem thermostatisierbaren Rührgefäss mit der halbmolaren Menge (bezogen auf Ketentrimer) Diethylenglykol versetzt und während 60 Min. auf 60 bis 65 °C erwärmt. Der Ketentrimergehalt reduzierte sich nach dieser Behandlung auf 3,1%.

Beispiel 3

0,1 kg Diketen-Rohprodukt und 9,22 g Octadecanol wurden innerhalb weniger Minuten auf 60 bis 65 °C aufgeheizt und 70 Min. bei dieser Temperatur belassen. In der abgekühlten Probe betrug der Ketentrimergehalt nun 1,8%. Dieser Wert entsprach einer Ketentrimergehaltssenkung um 2,5%.

Beispiel 4

In gleicher weise wie in Beispielen 1 bis 3 wurden in zwei weiteren Versuchen 0,1 kg Diketen-Rohprodukt mit 3,21 g Phenol bzw. 1,88 g Hydrochinon zur Reaktion gebracht. Die auf Raumtemperatur abgekühlten Proben wiesen folgende Ketentrimergehaltssenkungen auf:
Mit Phenol behandeltes Muster: 0,3% Ketentrimerabnahme nach der Behandlung.
Mit Hydrochinon behandeltes Muster: 1,8% Ketentrimerabnahme nach der Behandlung.

Beispiel 5

Ein Gemisch von 0,1 kg Roh-Diketen und 9,77 g 1-Octadecanthiol wurden innerhalb weniger Minuten auf 60 bis 65 °C aufgeheizt und für 0,5 Std. bei dieser Temperatur gehalten. Eine Temperaturerhöhung auf 95 bis 100 °C bewirkte eine Abnahme der Ketentrimergehaltes der Reaktionsmischung von 4,8 auf 3,3%.

Beispiel 6

Je 0,1 kg Roh-Diketen wurden in fünf verschiedenen Ansätzen mit 2,38 g Essigsäure, 8,74 g Palmitinsäure, 1,77 g Malonsäure, 2,01 g Bernsteinsäure, bzw. 4,16 g Benzoesäure bei Raumtemperatur vermischt. Die Mischungen wurden innerhalb weniger Minuten auf Reaktionstemperatur aufgeheizt. In allen Fällen war eine Abnahme des Ketentrimergehaltes zu beobachten:

| Carbonsäure | Reaktions-temperatur (°C) | Reaktions-zeit (h) | Ketentrimer-abnahme (%) |
|---|---|---|---|
| Essigsäure | 62 - 63 | 1,17 | 2,7 |
| Palmitinsäure | 62 | 1,17 | 2,8 |
| Malonsäure | 60 - 65 | 1,0 | 4,4 |
| Bernsteinsäure | 62 - 63 | 1,17 | 3,5 |
| Benzoesäure | 62 | 1,17 | 2,8 |

Beispiel 7

0,1 kg Diketenrohprodukt wurden bei Raumtemperatur mit 0,61 g Wasser vermischt und innerhalb weniger Minuten auf 60 bis 65°C erhitzt. Diese Temperatur des Reaktionsgemisches wurde für einen Zeitraum von 70 Min. beibehalten. Nach Abkühlung des Reaktionsgemisches auf 20°C resultierte eine Senkung des Ketentrimergehaltes des Diketenrohproduktes um 3,1%.

**Patentansprüche**

1. Verfahren zur Verminderung des Ketentrimeranteils im Roh-Diketen, dadurch gekennzeichnet, dass man Diketen mit einer dem Ketentrimer im Diketen entsprechenden Menge einer Verbindung der allgemeinen Formel

   $R(XH)_n$

   in welcher R = H, aliphatische Kohlenwasserstoffreste mit 1 bis 18 C-Atomen oder einen aromatischen Kohlenwasserstoffrest, X = Sauerstoff oder Schwefel, oder XH = eine COOH-Gruppe und n = 1 oder 2 bedeuten, versetzt, auf Temperaturen von 50 bis 130°C erwärmt und das Diketen abdestilliert.

2. Verfahren nach Patentanspruch 1, dadurch gekennzeichnet, dass man als Verbindung $R(XH)_n$ einen aliphatischen Alkohol mit 1 bis 18 C-Atomen verwendet.

3. Verfahren nach Patentanspruch 1, dadurch gekennzeichnet, dass man als Verbindung $R(XH)_n$ Phenol oder mehrwertige Phenole (n = 1 oder 2) verwendet.

4. Verfahren nach Patentanspruch 1, dadurch gekennzeichnet, dass man als Verbindung $R(XH)_n$ Carbonsäuren (n = 1 oder 2, XH = COOH) verwendet.

**Claims**

1. Process for reducing the amount of ketene trimer in a crude diketene, characterized in that the diketene is treated with a compound of the general formula

   $R(XH)_n$

   in which R = H, aliphatic hydrocarbon residues having 1 to 18 carbon atoms or an aromatic hydrocarbon residue, X = oxygen or sulfur, or XH = a COOH-group, and n= 1 or 2, in an amount corresponding to the ketene trimer in the diketene, heated to a temperature of from 50 to 130°C and the diketene is distilled off.

2. Process according to patent claim 1, characterized in that as compound $R(XH)_n$ an aliphatic alcohol having 1 to 18 carbon atoms is used.

3. Process according to patent claim 1, characterized in that as compound $R(XH)_n$ phenol or polyvalent phenols (n = 1 or 2) are used.

4. Process according to patent claim 1, characterized in that as compound $R(XH)_n$ carboxylic acids (n =1 or 2; XH = COOH) are used.

**Revendications**

1. Procédé pour abaisser la teneur en trimère de cétène dans le dicétène brut, caractérisé en ce qu'on ajoute au dicétène une quantité, correspondant au trimère de cétène contenu dans le dicétène, d'un composé de formule générale

   $R(XH)_n$

   dans laquelle R représente H, des restes hydrocarbonés aliphatiques de 1 à 18 atomes de carbone ou

un reste hydrocarboné aromatique, X représente l'oxygène ou le soufre, ou bien XH est un groupe COOH, et n est égal à 1 ou 2, en ce qu'on chauffe à des températures de 50 à 130 ° C et en ce qu'on distille le dicétène.

2. Procédé selon la revendication 1, caractérisé en ce qu'on utilise comme composé $R(XH)_n$ un alcool aliphatique de 1 à 18 atomes de carbone.

3. Procédé selon la revendication 1, caractérisé en ce qu'on utilise comme composé $R(XH)_n$ du phénol ou des polyphénols (n = 1 ou 2).

4. Procédé selon la revendication 1, caractérisé en ce qu'on utilise comme composé $R(XH)_n$ des acides carboxyliques (n = 1 ou 2, XH = COOH).